(19) Europäisches Patentamt
European Patent Office
Office européen des brevets

(11) EP 0 890 566 A1

(12) EUROPEAN PATENT APPLICATION

(43) Date of publication:
13.01.1999 Bulletin 1999/02

(21) Application number: 98810606.8

(22) Date of filing: 30.06.1998

(51) Int Cl.[6]: C07C 45/28, C07C 47/55, C07C 45/80, C07C 45/82, C25B 1/00

(84) Designated Contracting States:
AT BE CH CY DE DK ES FI FR GB GR IE IT LI LU MC NL PT SE
Designated Extension States:
AL LT LV MK RO SI

(30) Priority: 08.07.1997 GB 9714275

(71) Applicant: Ciba Specialty Chemicals Holding Inc.
4057 Basel (CH)

(72) Inventor: Weigmann, Reinhard
79500 Kandern (DE)

(54) **Oxidation process for the production of a chlorobenzaldehyde**

(57) There is provided a process for the production of a chlorobenzaldehyde comprising chemically oxidizing, in a first reaction vessel, a chlorotoluene using, as oxidizing agent, a trivalent manganese salt which has been generated, in a second reaction vessel, by electrochemical oxidation of a divalent manganese salt.

EP 0 890 566 A1

**Description**

The present invention relates to an oxidation process and, in particular, to a process for the oxidation of a chlorotoluene to a chlorobenzaldehyde.

According to the present invention, there is provided a process for the production of a chlorobenzaldehyde, preferably orthochlorobenzaldehyde (OCB) or parachlorobenzaldehyde (PCB), comprising chemically oxidizing, in a first reaction vessel, a chlorotoluene, preferably orthochlorotoluene (OCT) or parachlorotoluene (PCT), using, as oxidizing agent, a trivalent manganese salt which has been generated, in a second reaction vessel, by electrochemical oxidation of a divalent manganese salt.

The anion of the manganese salt may consist of any inorganic anion, such as the phosphate anion. The anion which gives minimal process problems in terms of cathodic and anodic stability, corrosion and aggression towards organic reactants or products, however, is the sulfate anion, so that the use of manganese sulfate, as oxidizing agent, is preferred in the process of the invention.

The chemical oxidation of (OCT) to (OCB) may be illustrated, in the preferred instance using manganese sulfate as oxidizing agent, by the following reaction scheme:

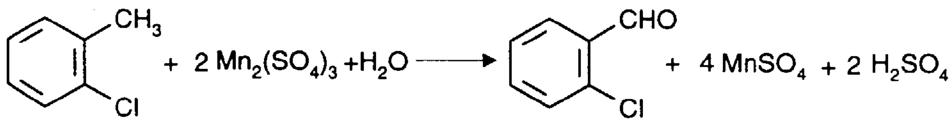


OCT OCB

In a secondary reaction, some of the OCT starting material may be oxidized to orthochlorobenzoic acid (OCBA) as follows:

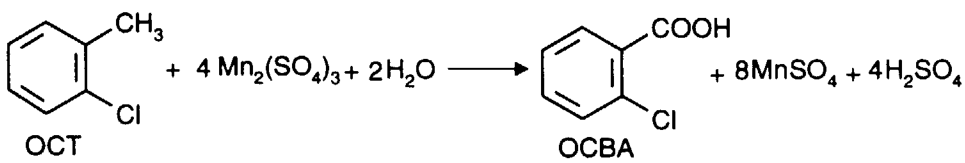


OCT OCBA

The electrochemical oxidation of the divalent manganese salt to the trivalent manganese salt may be illustrated, in the preferred instance using the sulfate, by reference to the following reaction scheme:

$$2\ MnSO_4 + H_2SO_4 \rightarrow Mn_2(SO_4)_3 + 2H^+ + 2e^-$$

The formation of the $Mn_2(SO_4)_3$ occurs at the anode. Secondary reactions which may take place at the anode are the formation of oxygen from water ($2\ H_2O \rightarrow 4\ H^+ + O_2 + 4\ e^-$), together with the hydration of OCT, OCB and OCBA to give $CO_2$ and $Cl_2$. A secondary reaction which may occur at the cathode is the formation of hydrogen ($2H^+ + 2e^- \rightarrow H_2$).

The electrochemical oxidation step is conveniently conducted in sulfuric acid, particularly in sulfuric acid having a concentration in the range of from 40 to 70%, more preferably in the range of from 50 to 60%. It is convenient to conduct the electrochemical oxidation step at an elevated temperature within the range of from 70 to 100°C., more preferably from 85 to 100°C. and, especially from 85 to 95°C.

The concentration of the total manganese salt during the electrochemical oxidation step may range from 2 to 6 moles per litre of electrolyte, a concentration of from 3 to 4 moles per litre of electrolyte being preferred.

The anode material and cathode material used in the electrochemical oxidation step are preferably the same and may be, e.g., vitreous graphite, lead, a lead alloy, platinum, zirconium coated with palladium or ruthenium, or tantalum coated with palladium or ruthenium. Particularly preferred are lead alloys containing silver.

The electrochemical oxidation step may be conveniently effected in a cell provided with an atmosphere of air. As already indicated, the potentially explosive gases hydrogen and oxygen are produced during the electrochemical oxidation step and the dilution with air helps to avoid any danger of explosion. The gases may be subjected to a wash to remove traces of chlorine.

Any convenient form of electrolytic cell constructed of inert material may be employed for the electrochemical

oxidation step. Preferably, the electrochemical oxidation step is conducted in a battery of electrolytic cells, which are preferably arranged in parallel. Preferred are cells of the bipolar type, especially those equipped with means to ensure good circulation and de-gasification.

The current density applied during the electrochemical oxidation step may range, for example, from 200 to 700 mA/$cm^2$, the range of from 300 to 500 mA/$cm^2$ being preferred.

The rate of conversion of divalent manganese into trivalent manganese obtained during the electrochemical oxidation step is high, conversion rates of up to 80% being readily achievable.

With respect to the separately conducted chemical oxidation step, it is preferably also conducted in sulfuric acid, particularly in sulfuric acid having a concentration in the range of from 40 to 70%, more preferably in the range of from 50 to 60%. It is convenient to conduct the chemical oxidation step at an elevated temperature within the range of from 80 to 110°C., and especially from 85 to 105°C.

The concentration of the total manganese salt during the chemical oxidation step may range from 2 to 6 moles per litre of oxidation reaction medium, a concentration of from 3 to 4 moles per litre of oxidation reaction medium being preferred. The oxidation reaction medium comprises an aqueous phase (sulfuric acid), solid inorganic manganese salts suspended in the aqueous phase and an organic liquid phase comprising OCT, OCB and OCBA. The use of an additional organic solvent, other than OCT, is unnecessary.

A conversion rate of chlorotoluene to chlorobenzaldehyde, preferably of OCT to OCB, of 89% is readily obtainable in the chemical oxidation step. In order to maximize the selectivity of the chlorotoluene to chlorobenzaldehyde conversion, and to minimize the formation of chlorobenzoic acid, is it preferred to interrupt the chlorotoluene to chlorobenzaldehyde conversion when a conversion rate of about 30 to 50% has been achieved. The chemical oxidation step consumes only chlorotoluene, some water and some electrical energy. Sole waste products are a minor amount of chlorobenzoic acid (about 3% on chlorotoluene), hydrogen and traces of chlorine.

The separation of the desired chlorobenzaldehyde from the reaction product mixture of the chemical oxidation step may be conducted by any conventional method. For example, a column extraction device may be employed. The organic phase may be subjected to an alkaline wash to remove traces of sulfuric acid and then distilled to separate the chlorobenzaldehyde product from unreacted chlorotoluene, which is then recycled into the chemical oxidation step.

The chlorobenzaldehydes, preferably OCB or PCB, obtained according to the process of the present invention are known compounds and find use, e.g., as intermediates for a wide range of chemical end-products, e.g. for perfumes, fluorescent whitening agents, dyestuffs and UV absorbers.

The following Example further illustrates the present invention.

<u>Example</u>

An electrochemical apparatus is set up which comprises:

a reactor having a capacity of 1.5 litres;
a circulation pump; and
an electrochemical cell having a lead anode and a lead cathode, the surface area of the anode being 1 square decimetre.

The apparatus is filled with 2,400 g of electrolyte composed of 745 g of $MnSO_4.H_2O$ and 1655 g of $H_2SO_4$ (55%). After electrolysis (electrochemical oxidation) at 85-90°C., over 6.5 hours, using a current of 36 amps and a cell voltage of 3.1 volts, there are obtained 1,704 g of $Mn_2(SO_4)_3$. The current efficiency is 55%.

The suspension of $Mn_2(SO_4)_3$ so obtained is introduced, with vigorous stirring, into a reactor having a capacity of 1.5 litres which contains 315 g of OCT and the mixture is thermostatized at 105 to 110°C. After a reaction time of 1.5 hours, the degree of conversion of $Mn^{3+}$ to $Mn^{2+}$ is greater than 95%.

After the reaction, the reaction mixture is transferred to a decantation flask in which the organic phase is separated from the inorganic phase, by means of hot decantation over 1 hour. The efficiency of this separation is 99.0%. The inorganic phase is then purified by stripping until the content of residual organic product is less than 400 ppm. The purified inorganic phase is then adjusted to the initial concentration of electrolyte and recycled to a subsequent electrolysis. The whole of the organic phase (317 g ) is washed with 100 g of NaOH (1%) and then distilled, under vacuum, whereby 112 g of OCB are separated from unreacted OCT and from residues. The rate of conversion of OCT into OCB is 81.1% and that of $Mn^{3+}$ into OCT is 84.4%.

Analogous results are obtained when using p-chlorotoluene (PCT) instead of OCT.

## Claims

1. A process for the production of a chlorobenzaldehyde comprising chemically oxidizing, in a first reaction vessel, a chlorotoluene using, as oxidizing agent, a trivalent manganese salt which has been generated, in a second reaction vessel, by electrochemical oxidation of a divalent manganese salt.

2. A process according to claim 1 in which the chlorotoluene is ortho- or para-chlorotoluene and the chlorobenzaldehyde produced is ortho- or para-chlorobenzaldehyde, respectively.

3. A process according to claim 2 in which the chlorotoluene is orthochlorotoluene and the chlorobenzaldehyde produced is orthochlorobenzaldehyde.

4. A process according to any of the preceding claims in which the anion of the manganese salt is the sulfate anion.

5. A process according to any of the preceding claims in which the electrochemical oxidation step is conducted in sulfuric acid.

6. A process according to claim 5 in which the electrochemical oxidation step is conducted in sulfuric acid having a concentration in the range of from 40 to 70%.

7. A process according to claim 5 in which the electrochemical oxidation step is conducted in sulfuric acid having a concentration in the range of from 50 to 60%.

8. A process according to any of the preceding claims in which the electrochemical oxidation step is effected at a temperature within the range of from 70 to 100°C.

9. A process according to claim 8 in which the electrochemical oxidation step is effected at a temperature within the range of from 85 to 95°C.

10. A process according to any of the preceding claims in which the concentration of the total manganese salt during the electrochemical oxidation step ranges from 2 to 6 moles per litre of electrolyte.

11. A process according to claim 9 in which the concentration of the total manganese salt during the electrochemical oxidation step ranges from 3 to 4 moles per litre of electrolyte.

12. A process according to any of the preceding claims in which the anode material and cathode material used in the electrochemical oxidation step are the same and are vitreous graphite, lead, a lead alloy, platinum, zirconium coated with palladium or ruthenium, or tantalum coated with palladium or ruthenium.

13. A process according to claim 12 in which the anode material and cathode material are lead alloys containing silver.

14. A process according to any of the preceding claims in which the the electrochemical oxidation step is effected in a cell provided with air dilution.

15. A process according to any of the preceding claims in which the the electrochemical oxidation step is effected in an electrolytic cell constructed of inert material.

16. A process according to claim 15 in which the electrochemical oxidation step is conducted in a battery of electrolytic cells, arranged in parallel.

17. A process according to claim 16 in which the cells are of the bipolar type and are equipped with means to ensure good circulation and de-gasification.

18. A process according to any of the preceding claims in which the current density applied during the electrochemical oxidation step ranges from 200 to 700 $mA/cm^2$.

19. A process according to claim 18 in which the current density applied during the electrochemical oxidation step ranges from 300 to 500 $mA/cm^2$.

**20.** A process according to any of the preceding claims in which the chemical oxidation step is conducted in sulfuric acid.

**21.** A process according to claim 20 in which the chemical oxidation step is conducted in sulfuric acid having a concentration in the range of from 40 to 70%.

**22.** A process according to claim 21 in which the chemical oxidation step is conducted in sulfuric acid having a concentration in the range of from 45 to 60%.

**23.** A process according to any of the preceding claims in which the chemical oxidation step is effected at a temperature within the range of from 80 to 110°C.

**24.** A process according to claim 23 in which the chemical oxidation step is effected at a temperature within the range of from 85 to 105°C.

**25.** A process according to any of the preceding claims in which the concentration of the total manganese salt during the chemical oxidation step ranges from 2 to 6 moles per litre of oxidation reaction medium.

**26.** A process according to claim 25 in which the concentration of the total manganese salt during the chemical oxidation step ranges from 3 to 4 moles per litre of oxidation reaction medium being preferred.

**27.** A process according to any of the preceding claims in which, in order to maximize the selectivity of the chlorotoluene to chlorobenzaldehyde conversion, and to minimize the formation of chlorobenzoic acid, the chlorotoluene to chlorobenzaldehyde conversion is interrupted when a conversion rate of about 30 to 50% has been achieved.

**28.** A process according to any of the preceding claims in which the separation of the desired chlorobenzaldehyde from the reaction product mixture of the chemical oxidation step is conducted using a column extraction device; and the organic phase is subjected to an alkaline wash to remove traces of sulfuric acid and is then distilled to separate the chlorobenzaldehyde product from unreacted chlorotoluene, which is then recycled into the chemical oxidation step.

European Patent Office

# EUROPEAN SEARCH REPORT

Application Number
EP 98 81 0606

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | GB 1 483 463 A (OXY METAL INDUSTRIES CORP) 17 August 1977<br>* the whole document * | 1-28 | C07C45/28<br>C07C47/55<br>C07C45/80<br>C07C45/82<br>C25B1/00 |
| Y | US 4 582 942 A (COMNINELLIS CHRISTOS ET AL) 15 April 1986<br>* the whole document * | 1-28 | |
| Y | GB 2 140 034 A (ELECTRICITY COUNCIL) 21 November 1984<br>* the whole document * | 1-28 | |
| A | S. CHIDAMBARAM ET AL.: "Electrolytically regenerated manganic sulphate for the oxidation of aromatic hydrocarbons : III. Oxidation of m-chlorotoluene, p-cymene and anethole"<br>JOURNAL OF THE ELECTROCHEMICAL SOCIETY OF INDIA,<br>vol. 17, no. 3, 1968, pages 95-96, XP002082877<br>* the whole document * | 1 | |
| A | CH. COMNINELLIS ET AL.: "Electrochemical production of manganic sulfate in concentrated H2SO4"<br>JOURNAL OF THE ELECTROCHEMICAL SOCIETY.,<br>vol. 129, no. 4, 1982, pages 749-752, XP002082878<br>MANCHESTER, NEW HAMPSHIRE US<br>* the whole document * | 5-9 | TECHNICAL FIELDS SEARCHED (Int.Cl.6)<br>C07C |

The present search report has been drawn up for all claims

| Place of search | Date of completion of the search | Examiner |
|---|---|---|
| THE HAGUE | 2 November 1998 | Bonnevalle, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)